# EUROPÄISCHE PATENTSCHRIFT

(11) **EP 1 969 133 B1**
(45) Veröffentlichungstag und Bekanntmachung des Hinweises auf die Patenterteilung: **01.09.2010**
(21) Anmeldenummer: 06841343.4
(22) Anmeldetag: 13.12.2006
(51) Int. Cl.: C12P 17/16, C07D 333/20, C07D 333/16

(54) **VERFAHREN ZUR HERSTELLUNG VON OPTISCH AKTIVEM (1S)-3-CHLOR-1-(2-THIENYL)-PROPAN-1-OL**
METHOD FOR PRODUCTION OF OPTICALLY ACTIVE (1S)-3-CHLORO-1-(2-THIENYL)-PROPAN-1-OL
PROCEDE DE PRODUCTION DE (1S)-3-CHLOR-1-(2-THIENYL)-PROPAN-1-OLE OPTIQUEMENT ACTIF

(30) Priorität: 23.12.2005 DE 102005062661
(43) Veröffentlichungstag der Anmeldung: 17.09.2008
(73) Patentinhaber: BASF SE, 67056 Ludwigshafen (DE)
(72) Erfinder: BREUER, Michael, 64285 Darmstadt (DE)
(74) Vertreter: Dörper, Thomas Michael
(86) Internationale Anmeldenummer: PCT/EP2006/069629
(87) Internationale Veröffentlichungsnummer: WO 2007/074060

(56) Entgegenhaltungen:
- WO-A-2005/033094
- HUMMEL W: "NEW ALCOHOL DEHYDROGENASES FOR THE SYNTHESIS OF CHIRAL COMPOUNDS" ADVANCES IN BIOCHEMICAL ENGINEERING, BIOTECHNOLOGY, SPRINGER, BERLIN, DE, Bd. 58, 1997, Seiten 145-184, XP000677754 ISSN: 0724-6145
- CHRISTIAN HEISS, ROBERT S. PHILLIPS: "Asymmetric reduction of ethynyl ketones and ethynylketoesters by secondary alcohol dehydrogenase from Thermoanaerobacter ethanolicus" J. CHEM. SOC., PERKIN TRANS I, 2000, Seiten 2821-2825, XP002433493

## Beschreibung

Die vorliegende Erfindung betrifft ein Verfahren zur Herstellung von optisch aktivem (1S)-3-Chlor-1-(2-thienyl)-propan-1-ol durch enzymatische Reduktion.

### Stand der Technik:

(1S)-3-Chlor-1-(2-thienyl)-propan-1-ol ist eine direkte Vorstufe für die Herstellung von (1S)-3-Methylamino-1-(2-thienyl)-propan-1-ol ("Duloxetinealkohol"), der wiederum Baustein in der Duloxetine-Synthese ist.. Duloxetine® ist ein Pharmawirkstoff, der sich momentan in der Zulassung befindet und im Indikationsgebiet Depression und Inkontinenz eingesetzt werden soll.

EP-B-0273658 beschreibt ein Verfahren zur Herstellung der korrespondierenden Base von Duloxetine durch Umsetzung von 2-Acetylthiophen in einer Mannich-Reaktion mit Formaldehyd und Dimethylamin, Reduktion der Ketogruppe der dabei erhaltenen Mannich-Base zum racemischen (S)-3-N,N-Dimethylamino-1-(thien-2-yl)propan-1-ol, Veretherung der Alkoholfunktion mit Naphthylfluorid und schließlich Umwandlung der Dimethylamino-Gruppe in eine Methylamino-Funktion. Das gewünschte Enantiomer des Naphtylethers erhält man durch Einsatz chiraler Ausgangsmaterialien oder durch Racemattrennung auf der Stufe des Endprodukts, beispielsweise über die Salze mit optisch aktiven Säuren oder Chromatographie an einer chiralen stationären Phase.

US-5,362,886 beschreibt ein analoges Verfahren, bei dem das nach Reduktion der Ketogruppe erhaltene racemische Propanol mit S-Mandelsäure versetzt wird. Das hierbei erhaltene S-Enantiomer des Alkohols wird in die nachfolgenden Reaktionsstufen eingesetzt.

EP-A-0457559 beschreibt ebenfalls ein der EP-B-0273658 analoges Verfahren. Hierbei wird die Ketogruppe der Mannich-Base mit dem asymmetrischen Reduktionssystem LAH-Icb (Lithiumaluminiumhydrid-[(2R,2S)-(-)-4-Dimethylamino-1,2-diphenyl-3-methyl-2-butanol]) zum Alkohol in Form des S-Enantiomers reduziert. Nachteilig hierbei ist neben den Kosten die Empfindlichkeit des Reduktionssystems LAH-Icb, das nur wenige Minuten stabil ist.

W. J. Wheeler und F. Kuo beschreiben in Journal of Labelled Compounds and Radiopharmaceuticals, Band XXXVI, Nr. 3, Seite 213 bis 223 ein Verfahren zur Herstellung von Duloxetine. Hierzu wird Thiophen-2-carbonsäurechlorid in einer Stille-Kopplung mit Vinyl-tri-n-butylstannan in Gegenwart katalytischer Mengen Benzylchlor-bis(triphenylphosphin)palladium(II) in DMPU (Dimethylpropylenharnstoff) zu 1-(Thien-2-yl)-propenon der Formel (V) 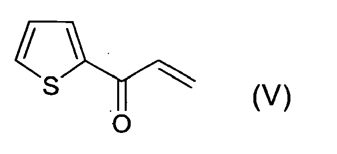 umgesetzt, welches anschließend durch Behandlung mit Chlorwasserstoff in 3-Chlor-1-(thien-2-yl)-propan-1-on der Formel (VI) 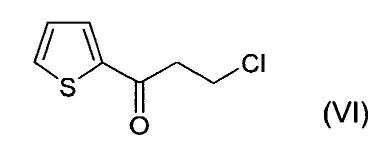 überführt wird. Das so erhaltene Chlorpropanon wird anschließend unter Verwendung eines chiralen Oxazaborylidins und BH₃ zu (S)-3-Chlor-1-(thien-2-yl)-propan-1-ol der Formel (VII) 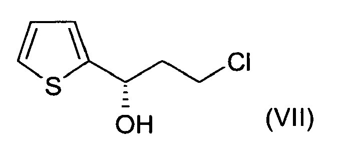 reduziert. Der so erhaltene Alkohol wird durch sukzessive Umsetzung mit Natriumiodid und anschließend mit Methylamin in (S)-3-Methylamino-1-(thien-2-yl)-propan-1-ol überführt. Durch nachfolgende sukzessive Umsetzung mit Natriumhydrid, 1-Fluornaphthalin und Chlorwasserstoff erhält man Duloxetine in Form des Hydrochlorids. 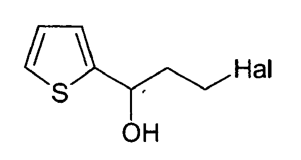 Hal = Halogen

T. Stillger et al. beschreiben in Chemie Ingenieur Technik (74) Seiten 1035-1039, 2002 substratgekoppelte Cofaktorregenerierungsverfahren zur enzymatischen enantioselektiven Reduktion von 5-Oxohexansäureethylester zu (S)-5-Hydroxyhexansäureethylester.

### Kurze Beschreibung der Erfindung:

Der Erfindung lag daher die Aufgabe zugrunde, einen Weg zur stereospezifischen Reduktion von optisch aktiven (1S)-3-Chlor-1-(2-thienyl)-propan-1-ol, zu finden, wobei das Reaktionsverfahren auf kostengünstigem Weg möglichst quantitativ zum Produkt führen sollte.

Diese Aufgabe wurde durch den überraschenden Befund gelöst, dass Enzyme mit Dehydrogenase-Aktivität aus der Gattung Thermoanaerobacter herstellbar sind die zur stereospezifischen Katalyse der obigen Reaktion befähigt sind.

### Detaillierte Beschreibung der Erfindung:

Gegenstand der Erfindung ist ein Verfahren zur Herstellung von optisch aktiven (1S)-3-Chlor-1-(2-thienyl)-propan-1-ol der Formel I 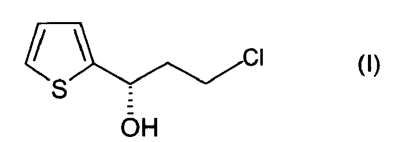 wobei in einem 3-Chlor-1-(2-thienyl)-propan-1-on der Formel II 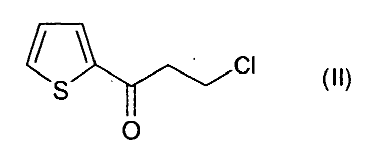 enthaltenden Medium diese Verbindung zur Verbindung der Formel I durch eine Alkoholdehydrogenase aus der Gattung Thermoanaerobacter reduziert wird, und man das in im Wesentlichen enantiomerenreiner Form gebildete Produkt isoliert.

Die erfindungsgemäss verwendeten Enzyme mit Dehydrogenase-Aktivität können im erfindungsgemäßen Verfahren als freies oder immobilisiertes Enzym verwendet werden.

Das erfindungsgemäße Verfahren wird vorteilhaft bei einer Temperatur zwischen 0 °C bis 95 °C, bevorzugt zwischen 10 °C bis 85 °C, besonders bevorzugt zwischen 15 °C bis 75 °C durchgeführt.

Der pH-Wert im erfindungsgemäßen Verfahren wird vorteilhaft zwischen pH 4 und 12, bevorzugt zwischen pH 4,5 und 9, besonders bevorzugt zwischen pH 5 und 8 gehalten.

Unter enantiomerenreinen bzw. chiralen Produkten bzw. optisch aktiven Alkoholen sind im erfindungsgemäßen Verfahren Enantiomere zu verstehen, die eine Enantiomerenanreicherung zeigen. Bevorzugt werden im Verfahren Enantiomerenreinheiten von mindestens 70 %ee, bevorzugt von min. 80 %ee, besonders bevorzugt von min. 90 %ee, ganz besonders bevorzugt min. 98 %ee erreicht.

Für das erfindungsgemäße Verfahren können wachsende Zellen verwendet werden, die die erfindungsgemäßen Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakt sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Werden für das erfindungsgemäße Verfahren freie Organismen oder Enzyme verwendet, so werden diese vor der Extraktion zweckmäßigerweise abgetrennt beispielsweise über eine Filtration oder Zentrifugation.

Das im erfindungsgemäßen Verfahren hergestellte Produkt (1S)-3-Chlor-1-(2-thienyl)-propan-1-ol, lässt sich vorteilhaft aus der wässrigen Reaktionslösung über Extraktion oder Destillation gewinnen. Die Extraktion kann zur Erhöhung der Ausbeute mehrfach wiederholt werden. Beispiele für geeignete Extraktionsmittel sind Lösungsmittel, wie Toluol, Methylenchlorid, Butylacetat, Diisopropylether, Benzol, MTBE oder Essigester, ohne darauf beschränkt zu sein.

Alternativ kann das im erfindungsgemäßen Verfahren hergestellte Produkt (1S)-3-Chlor-1-(2-thienyl)-propan-1-ol, sich vorteilhaft aus der organischen Phase der Reaktionslösung über Extraktion oder Destillation oder/und Kristallisation gewinnen. Die Extraktion kann zur Erhöhung der Ausbeute mehrfach wiederholt werden. Beispiele für geeignete Extraktionsmittel sind Lösungsmittel, wie Toluol, Methylenchlorid, Butylacetat, Diisopropylether, Benzol, MTBE oder Essigester, ohne darauf beschränkt zu sein.

Nach Einengen der organischen Phase können die Produkte in der Regel in guten chemischen Reinheiten, das heißt größer 80 % chemische Reinheit, gewonnen werden. Nach Extraktion kann die organische Phase mit dem Produkt aber auch nur zum Teil eingeengt werden und das Produkt auskristallisiert werden. Dazu wird die Lösung vorteilhaft auf eine Temperatur von 0 °C bis 10 °C abgekühlt. Die Kristallisation kann auch direkt aus der organischen Lösung oder aus einer wässrigen Lösung erfolgen. Das auskristallisierte Produkt kann nochmals im gleichen oder in einem anderen Lösungsmittel zur erneuten Kristallisation aufgenommen werden und nochmals kristallisiert werden. Durch die anschließende vorteilhafte mindestens einmalig durchgeführte Kristallisation kann die Enantiomerenreinheit des Produktes falls erforderlich weiter gesteigert werden.

Bei den genannten Aufarbeitungsarten lässt sich das Produkt des erfindungsgemäßen Verfahrens in Ausbeuten von 60 bis 100 %, bevorzugt von 80 bis 100 %, besonders bevorzugt von 90 bis 100 %, bezogen auf das für die Reaktion eingesetzte Substrat, wie z.B. von 3-Chlor-1-(2-thienyl)-propan-1-on, isolieren. Das isolierte Produkt zeichnet sich durch eine hohe chemische Reinheit von > 90 %, bevorzugt > 95 % besonders bevorzugt von > 98 % aus. Weiterhin haben die Produkt eine hohe Enantiomerenreinheit, die vorteilhaft falls erforderlich durch die Kristallisation weiter gesteigert werden kann.

Das erfindungsgemäße Verfahren kann batchweise, semi-batchweise oder kontinuierlich betrieben werden.

Die Durchführung des Verfahrens kann vorteilhafterweise in Bioreaktoren erfolgen, wie z.B. beschrieben in Biotechnology, Band 3, 2. Auflage, Rehm et al Hrsg., (1993) insbesondere Kapitel II.

Die obige Beschreibung und die nachstehenden Beispiele dienen nur der Verdeutlichung der Erfindung. Die für den Fachmann offensichtlichen, zahlreich möglichen Abwandlungen sind erfindungsgemäß ebenfalls umfasst.

Der Vorteil des erfindungsgemäßen Verfahrens liegt in der besonders hohen Ausbeute des optisch aktiven Alkanols der Formel (I) bzw. der nahezu quantitativen Umwandlung von Alkanon (II).

Bei den enzymatischen Reduktion mittels Alkoholdehydrogenase werden Kofaktoren benötigt, die im Verlauf der Reaktion verbraucht, d.h. oxidiert werden. Bevorzugter Kofaktor ist NADPH, das zu NADP oxidiert wird.

Eine bevorzugte Ausführungsform der Erfindung regeneriert die Kofaktoren, indem sie mit einer gleichzeitig ablaufenden Oxidationsreaktion gekoppelt werden. Besonders bevorzugt ist hierfür das System Alkanol/Keton, insbesonders Isopropanol /Aceton, da dieses ebenfalls von der Alkoholdehydrogenase katalysiert wird.

Das bei der Reduktion der Verbindung (II) zu Verbindung (I) verbrauchte NADPH wird durch die enzymatische Oxidation von Isopropanol zu Aceton wieder regeneriert. Hierbei ist dafür zu sorgen, dass ausreichen Isopropanol (sg. Opferalkohol) zur Verfügung steht, und dass keine zu hohen Konzentrationen an evt. das Enzym schädigendem Aceton entstehen.

### Experimenteller Teil:

### Enzym

Die im folgenden verwendete Alkoholdehydrogenase aus *Thermoanaerobacter* spec. (im folgenden abgekürzt mit ADH-T) ist kommerziell erhältlich und wurde von der Firma Jülich Fine Chemicals bezogen (Best.Nr..90112610, Alcohol dehydrogenase T from *Thermoanaerobacter* spec). Die ADH-T wurde ohne weitere Reinigung verwendet.

### Inkubation mit NADPH

In einem mL Puffer 50mM NaH₂PO₄ (pH5) wurden 10µmol NADPH, 10µmol 3-Chlor-1-(thien-2-yl)-propan-1-on und ADH-T (0.58mg Protein) bei 30°C inkubiert. Nach 60min wurde die Reaktion durch Zugabe von HCl_{konz}. beendet und das denaturierte Protein mittels Zentrifugation entfernt. Der Überstand nach Zentrifugation wurde chromatographisch untersucht. Anhand der Peakflächen von Edukt und Produkt kann die Aktivität des Enzyms berechnet werden. Durch Verwendung chiralen Chromatographie-Materials ist eine Unterscheidung der beiden Enantiomere von (1*S*)-3-Chlor-1-(thien-2-yl)-propan-1-ol möglich.

### Inkubation mit NADP und iso-Propanol (Kofaktorregenerierung)

Der Kofaktor NADPH wird während der Reduktion verbraucht. Es ist bekannt, dass die ADH-T die Oxidation von i-Propanol zu Aceton durchführt und somit den verbrauchten Kofaktor regenerieren kann. Diese Fahrweise ist vorteilhaft, weil NADPH nur in katalytischen Mengen der Reaktionsmischung zugesetzt werden muss.

In einem mL Puffer (50mM NaH₂PO₄ (pH5) mit 10% 2-Propanol) wurden 0,2µmol NADP, 10µmol 3-Chlor-1-(thien-2-yl)-propan-1-on und ADH-T (0.58mg Protein) bei 30°C inkubiert. Nach 60min wurde die Reaktion durch Zugabe von HCl_{konz.} beendet und das denaturierte Protein mittels Zentrifugation entfernt. Aufarbeitung und Analytik erfolgen wie oben beschrieben.

### Analytik von (1S)-3-Chlor-1-(thien-2-yl)-propan-1-ol

Die Konzentration von 3-Chlor-1-(thien-2-yl)-propan-1-on und 3-Chlor-1-(thien-2-yl)-propan-1-ol lassen sich mittels HPLC bestimmen. Je nach Wahl der stationären und mobilen Phase lassen sich neben der Konzentration auch ee-Wert bestimmen.

### a) achirale Analytik

Die Quantifizierung der Umsetzung wurde mit folgendem System durchgeführt:
stationäre Phase: Chromoltih SpeedROD RP18, 50*4, 6µm, Merck (Darmstadt) tem-periert auf 45°C

| | |
|---|---|
| mobilePhase: | Laufmittel A: 10mM KH2PO4, pH 2.5 |
| Laufmittel B: Acetonitril | |
| Gradient: 0-0.5 min, 35%B; 0.5-1.0 min 35 auf 80%B; 1.0-1.2 min 80%B; 1.2-1.3 min 80% - 35%B; 1.3-2.0 min 35%B; | |
| Flussrate: | 1.5 ml/min |
| Detektion: | UV-Detektion bei 230 und 260nm |
| Retentionszeiten: | 3-Chlor-1-(thien-2-yl)-propan-1-on: ca. 1.6 min |
| 3-Chlor-1-(thien-2-yl)-propan-1-ol: ca. 1.3 min | |

Mit authentischem Material wird eine Eichreihe erstellt, anhand derer die Konzentration unbekannter Proben bestimmt werden kann.

### b) chirale Analytik

| | |
|---|---|
| stationäre Phase: | Chiracel OD-H, 250*4, 6µm, Daicel, temperiert auf 40°C |
| mobilePhase: | Laufmittel A: n-Hexan |
| Laufmittel B: iso-Propanol | |
| isokratisch mit 2.5% B | |
| Flussrate: | 1.0 ml/min |
| Detektion: | UV-Detektion bei 230 und 260nm |
| Retentionszeiten: | 3-Chlor-1-(thien-2-yl)-propan-1-on: ca. 9.5 min |
| (1S)-3-Chlor-1-(thien-2-yl)-propan-1-ol: ca. 16.6 min | |
| (1R)-3-Chlor-1-(thien-2-yl)-propan-1-ol: ca. 18.3 min | |

Mit authentischem Material wird eine Eichreihe erstellt, anhand derer die Konzentration unbekannter Proben bestimmt werden kann.

### Ergebnis

### Umsetzung ohne Kofaktorregenerierung

Nach 60minütiger Inkubation konnten 2.1 mM (1*S*)-3-Chlor-1-(thien-2-yl)-propan-1-ol im Reaktionsansatz bestimmt werden. Die spezifische Aktivität der ADH-T liegt beim Start der Reaktion bei 98 U/g Protein. Der entstandene Alkohol weist einen ee-Wert von 95% auf.

### Umsetzung mit Kofaktorregenerierung

Auch in diesem System ist die ADH-T in der Lage durch Substratkopplung sowohl Reduktions- als auch Oxidationsreaktion zu katalysieren. Nach einstündiger Inkubation wurden allerdings nur 0,7 mM (1*S*)-3-Chlor-1-(thien-2-yl)-propan-1-ol im Reaktionsansatz gefunden. Auch die spezifische Aktivität des Enzym ist zu Beginn der Reaktion mit 23 U/g Protein sehr gering. Der entstandene Alkohol weist einen ee-Wert von 94% auf. Die geringere Aktivität lässt sich dadurch erklären, dass vermutlich nicht die optimalen Bedingungen für die iso-Propanol-Oxidation vorgelegen haben. Möglicherweise ist auch die Gleichgewichtslage der Gesamtreaktion ungünstig.

Alternativ kann die Kofaktorregenerierung auch die Zugabe eines Hilfsenzyms wie zum Beispiel einer Glucosedehydrogenase oder Formiatdehydrogenase erfolgen. In diesem Fall ist anstelle iso-Propanol ein anderes Zweitsubstrat wie Glucose bzw. Formiat notwendig. Glucosedehydrogenase oxidiert Glucose zur Gluconsäure und reduziert dabei den Kofaktor während die Kofaktorregenerierung mit Formiatdehydrogenase zu CO₂ als Endprodukt führt.

## Patentansprüche

1. Verfahren zur Herstellung von optisch aktiven (1S)-3-Chlor-1-(2-thienyl)-propan-1-ol der Formel I 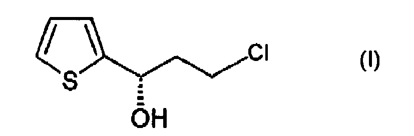 wobei in einem 3-Chlor-1-(2-thienyl)-propan-1-on der Formel II 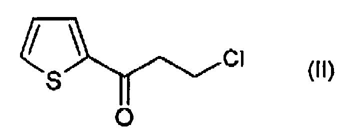 enthaltenden Medium diese Verbindung zur Verbindung der Formel I durch eine Alkoholdehydrogenase aus Thermoanaerobacter spec. reduziert wird, und man das in im Wesentlichen enantiomerenreiner Form gebildete Produkt isoliert.

2. Verfahren nach Anspruch 1, **dadurch gekennzeichnet, dass** man NADPH als Kofaktor bei der Reduzierung einsetzt.

3. Verfahren nach Anspruch 2, **dadurch gekennzeichnet, dass** man bei der Reduktion oxidierte Kofaktoren regeneriert.

4. Verfahren nach Anspruch 3, **dadurch gekennzeichnet, dass** man die Regenerierung der Kofaktoren durch die enzymatische Oxidation von Isopropanol zu Aceton durchführt.

## Claims

1. A process for preparing optically active (1S)-3-chloro-1-(2-thienyl)propan-1-ol of the formula I 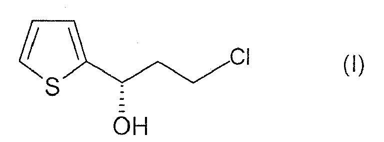 by, in a medium comprising 3-chloro-1-(2-thienyl)propan-1-one of the formula II 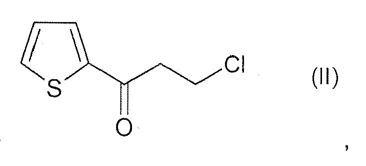 reducing this compound to the compound of the formula I by means of an alcohol dehydrogenase from Thermoanaerobacter spec., and isolating the product formed in substantially enantiomerically pure form.

2. The process according to claim 1, wherein NADPH is used as a cofactor in the reduction.

3. The process according to claim 2, wherein oxidized cofactors are regenerated in the reduction.

4. The process according to claim 3, wherein the regeneration of the cofactors is carried out by the enzymatic oxidation of isopropanol to acetone.

## Revendications

1. Procédé pour la préparation de (1S)-3-chloro-1-(2-thiényl)-propan-1-ol optiquement actif de formule I 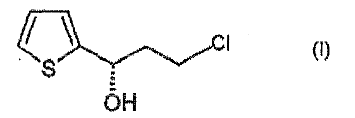 où, dans un milieu contenant de la 3-chloro-1-(2-thiényl)-propan-1-one de formule II 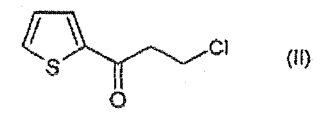 ce composé est réduit en composé de formule I par une alcool-déshydrogénase de Thermoanaerobacter spec., et on isole le produit formé sous une forme essentiellement énantiomère pure.

2. Procédé selon la revendication 1, **caractérisé en ce qu'**on utilise du NADPH comme cofacteur lors de la réduction.

3. Procédé selon la revendication 2, **caractérisé en ce qu'**on régénère les cofacteurs oxydés lors de la réduction.

4. Procédé selon la revendication 3, **caractérisé en ce qu'**on réalise la régénération des cofacteurs par l'oxydation enzymatique d'isopropanol en acétone.
